(19) Europäisches Patentamt
European Patent Office
Office européen des brevets

(11) EP 0 804 241 B1

# (12) FASCICULE DE BREVET EUROPEEN

(45) Date de publication et mention de la délivrance du brevet:
**23.10.2002 Bulletin 2002/43**

(21) Numéro de dépôt: **96901410.9**

(22) Date de dépôt: **18.01.1996**

(51) Int Cl.[7]: **A61K 47/14**, A61K 47/44, A01N 25/02

(86) Numéro de dépôt international:
**PCT/FR96/00080**

(87) Numéro de publication internationale:
**WO 96/022109 (25.07.1996 Gazette 1996/34)**

(54) **UTILISATION D'ESTERS D'ACIDES GRAS ETHOXYLES COMME COMPOSANTS AUTO-EMULSIONNABLES**

VERWENDUNG ETHOXILIERTER FETTSÄUREESTER ALS SELBSTEMULGIERENDE KOMPONENTEN

UTILIZATION OF ETHOXYLATED FATTY ACID ESTERS AS SELF-EMULSIFIABLE COMPOUNDS

(84) Etats contractants désignés:
**BE CH DE DK ES FR GB IT LI NL SE**

(30) Priorité: **18.01.1995 FR 9500497**

(43) Date de publication de la demande:
**05.11.1997 Bulletin 1997/45**

(73) Titulaire: **SOCIETE D'EXPLOITATION DE PRODUITS POUR LES INDUSTRIES CHIMIQUES, S.E.P.P.I.C.**
**75321 Paris Cédex 07 (FR)**

(72) Inventeur: **TROUVE, Gérard**
**F-81100 Castres (FR)**

(74) Mandataire: **Hubert, Philippe**
**Cabinet Beau de Loménie**
**158, rue de l'Université**
**75340 Paris Cédex 07 (FR)**

(56) Documents cités:
**EP-A- 0 292 050**
**WO-A-95/08983**
**BE-A- 660 601**
**FR-A- 2 534 923**
**US-A- 3 539 518**

- **SEIFEN-ÖLE-FETTE-WACHSE, vol. 111, no. 2, Février 1985, AUGSBURG (DE), page 51 XP002000689 V. MARTIN: "ETHOXYLIERTE TRIGLYCERIDE ALS RÜCKFETTER UND SOLUBILISATOREN"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 804 241 B1

## Description

La présente invention concerne généralement l'utilisation d'esters d'acides gras éthoxylés comme composants auto-émulsionnables, notamment utiles pour la préparation de produits de traitement phytosanitaire ou de médicaments à usage vétérinaire ou humain.
On sait qu'une émulsion est un mélange d'eau et d'huile stabilisé par des agents tensioactifs.
La dispersion de l'huile dans l'eau (ou de l'eau dans l'huile) sous forme de gouttelettes suffisamment fines pour obtenir une émulsion stable nécessite un apport d'énergie souvent considérable.
Par ailleurs, on appelle composition auto-émulsionnable, toute préparation huileuse capable de former une émulsion stable avec une phase aqueuse, pratiquement sans apport d'énergie, par exemple par dispersion dans la phase aqueuse par agitation mécanique lente.
Les compositions auto-émulsionnables sont particulièrement appréciées chaque fois que des mélanges avec une phase aqueuse, généralement de l'eau, doivent être réalisés sans faire appel à des moyens d'agitation performants.
Il en est ainsi notamment :

- dans le cadre d'applications domestiques, par exemple pour la préparation de produits d'entretien ménagers ou de produits de jardinage ;
- dans le cadre d'applications agricoles, par exemple pour la préparation de produits phytosanitaires destinés à être stockés dans des citernes ou autres conteneurs ;
- dans le domaine pharmaceutique, par exemple pour la préparation de médicaments faite extemporanément.

Des préparations auto-émulsionnables ont été décrites depuis très longtemps dans l'état de la technique, notamment dans le domaine agro-chimique.
Ces préparations sont généralement constituées d'huiles minérales ou de coupes pétrolières additivées d'agents tensioactifs peu onéreux tels que en particulier les alkyls phénols éthoxylés.
Cependant de telles préparations sont faiblement biodégradables et représentent donc un danger potentiel pour l'environnement, ce qui en a limité le développement ces dernières années.
On s'est donc orienté récemment vers la recherche de produits de substitution des huiles pétrolières et l'on a préconisé à cet effet l'utilisation d'huiles biodégradables comme en particulier les triglycérides ou les esters méthyliques d'acides gras.
Cependant, ces huiles sont beaucoup plus difficiles à émulsionner que les huiles minérales.
Par ailleurs, il a également été envisagé de remplacer les alkyls phénols éthoxylés par des agents tensioactifs biodégradables, mais ceci s'est avéré extrêmement difficile pour des questions de coût et d'efficacité.
Dans ce contexte, la présente invention a pour but de résoudre le problème technique consistant en la fourniture d'une nouvelle composition auto-émulsionnable notamment utile pour la préparation de produits de traitement phytosanitaires ou de médicaments à usage vétérinaire ou humain, dont la mise en oeuvre est aisée et qui présente un caractère biodégradable suffisant pour répondre au souci du respect de l'environnement.
Il a été découvert, et ceci constitue le fondement de la présente invention, que certains esters obtenus par réaction entre des acides gras, des alcools légers ou des polyols et de l'oxyde d'éthylène constituent des composants auto-émulsionnables sans l'aide d'aucun autre agent tensioactif, et sont biodégradables et capables de dissoudre des principes actifs peu ou pas solubles dans l'eau.
Ainsi, selon un premier aspect, la présente demande vise à couvrir l'utilisation d'esters d'acides gras éthoxylés répondant à l'une des formules suivantes :

(I) 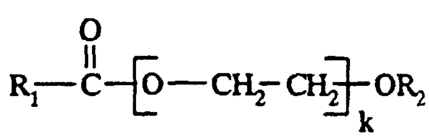

(II) 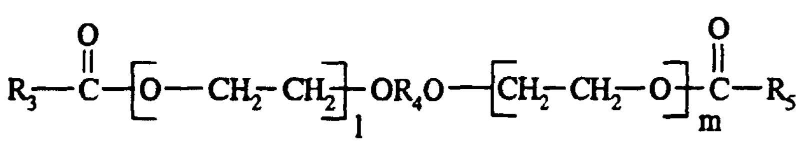

(III) 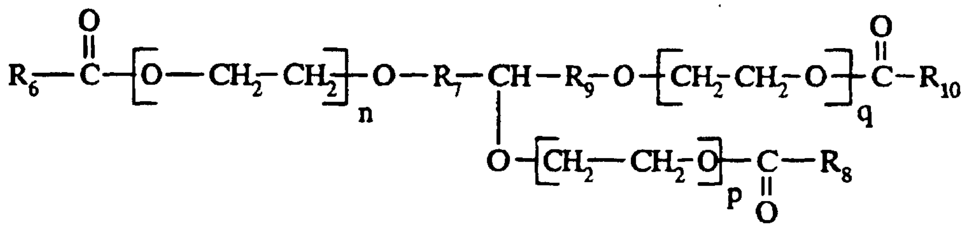

dans lesquelles :

- $R_1$, $R_3$, $R_5$, $R_6$, $R_8$ et $R_{10}$ représentent une chaîne hydrocarbonée, linéaire ou ramifiée, saturée ou insaturée ayant de 5 à 30 atomes de carbones ;
- $R_2$, $R_4$, $R_7$ et $R_9$ représentent une chaîne hydrocarbonée, linéaire ou ramifiée, saturée ou insaturée ayant de 1 à 5 atomes de carbone ;

le nombre total de molécules d'oxyde d'éthylène respectivement représenté dans les formules I, II et III précitées par k, l+m, n+p+q étant un nombre entier tel que la valeur HLB (balance hydrophile-lipophile) desdits composés soit comprise entre 4 et 10, de préférence entre 5 et 9; et de préférence encore voisine de 5.

comme composants auto-émulsionnables notamment utiles pour la préparation de produits phytosanitaires ou de médicaments à usage vétérinaire ou humain.

Avantageusement, les produits suivants ainsi que leurs mélanges pourront être utilisés conformément à la présente invention :

- Esters d'acides gras éthoxylés répondant à la formule 1 précitée dans laquelle $R_1$ est choisi parmi les restes des acides palmitique, stéarique, ricinoléique, oléique, linoléique et linolénique ; $R_2$ représente un radical méthyl et k est un nombre entier compris entre 1 et 5, de préférence égal à 2 ;
- Esters d'acides gras éthoxylés répondant à la formule III précitée dans laquelle :
  - . $R_6$, $R_8$ et $R_{10}$ représentent des chaînes hydrocarbonées correspondant aux chaînes grasses d'une huile végétale ;
  - . $R_7$ et $R_9$ représentent un groupe méthylène $CH_2$ ;
  - . n, p, q présentent des nombres entiers tels que leur somme soit comprise entre 3 et 30.
- Esters d'acides gras éthoxylés répondant à la formule III précitée dans laquelle :
  - . $R_6$, $R_8$ et $R_{10}$ représentent des chaînes hydrocarbonées ayant de 16 à 22 atomes de carbones correspondant aux chaînes grasses de l'huile de colza ;
  - . $R_7$ et $R_9$ représentent un groupe méthylène $CH_2$ ;
  - . n, p, q présentent des nombres entiers tels que leur somme soit comprise entre 3 et 30, et de préférence égale à 20;
- Esters d'acides gras éthoxylés répondant à la formule III précitée dans laquelle :
  - . $R_6$, $R_8$ et $R_{10}$ représentent des chaînes hydrocarbonées correspondant aux chaînes grasses de l'huile de ricin ;
  - . $R_7$ et $R_9$ représentent un radical méthylène $CH_2$ ;
  - . n, p et q représentent des nombres entiers tels que leur somme soit comprise entre 5 et 7.

Dans le cadre de la présente description et des revendications, on entend par chaîne hydrocarbonée toute chaîne constituée exclusivement d'atomes de carbone et d'atomes d'hydrogène.

Des chaînes alkyles, alcényles ou alcynyles constituent des exemples de telles chaînes hydrocarbonées.

La balance hydrophile-lipophile (designée également HLB) est définie par la formule suivante :

$$HLB = 20(1-\frac{IS}{IA})$$

dans laquelle :

- IS représente l'indice de saponification du produit mesuré selon la nonne NFT 60206 ; et
- IA représente l'indice d'acide de l'acide utilisé pour la fabrication du produit mésuré selon la norme NFT 60204.

Les composés les plus intéressants dans le cadre de la présente invention sont ceux liquides à température ambiante.

Le mélange de plusieurs produits répondant aux formules I, II et III précitées peut être avantageusement utilisé pour la préparation de compositions auto-émulsionnables spécifiques présentant des caractéristiques particulières de densité, de viscosité ou de point de figeage.

L'ajout de solvants biodégradables, miscibles avec ces produits, tels que des triglycérides, des glycols, des esters ou cétones légers est également envisageable.

Par esters ou cétones légers, on désigne des esters ou des cétones ayant des propriétés de solvants reconnus et obtenus généralement par condensation d'un acide gras court (moins de 10 atomes de carbone) avec un alcool de moins de 10 atomes de carbones.

La méthyl-isobutyl cétone, la méthyl éthyl cétone, l'acétate d'éthyle, l'acétate d'amyle, l'acétate d'isoamyle en sont des exemples.

A titre d'exemple de solvant biodégradable préféré, on peut citer le propylène-glycol, la glycérine et le triacétate de glycérol.

Ces solvants pourront être présents dans la composition auto-émulsionnable en des quantités variant de 0 à environ 50% en poids par rapport au poids total de la composition.

Des esters d'acides gras éthoxylés présentant une structure chimique très proche de celle des composés de formules I, II et III précitées ont été décrits dans la littérature, par exemple dans les documents US 2,678,935 ; US 3,539,518 ; US 4,022,808 ; GB 1,050,497.

Cependant, ces documents antérieurs ne comportent aucune indication permettant à l'homme de métier de déduire que certains esters d'acides gras éthoxylés répondant aux formules I à III précitées constitueraient une phase auto-émulsionnable.

Les composés utilisés dans le cadre de la présente invention peuvent être facilement préparés par des procédés de synthèse semblables à ceux décrits dans l'état de la technique précitée.

D'une façon générale, ces composés peuvent être obtenus :

- soit par estérification d'alcools éthoxylés comme décrit dans le document US 3,539,518;
- soit, de manière préférée, par éthoxylation d'esters répondant aux formules générales:

(I') 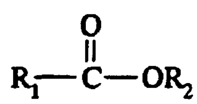

(II') 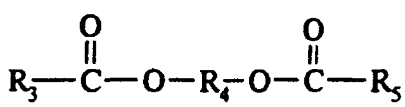

(III') 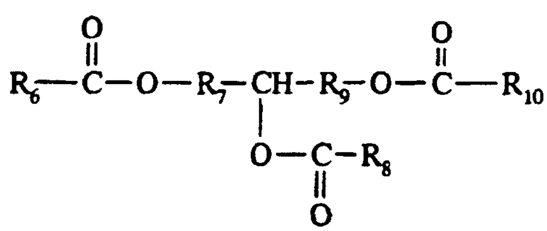

dans lesquelles, $R_1$, $R_2$, $R_3$, $R_4$, $R_5$, $R_6$, $R_7$, $R_8$, $R_9$, $R_{10}$ sont tels que définis précédemment, comme décrit dans le document GB 1,050,497.

La réaction d'éthoxylation sera généralement réalisée par réaction de l'oxyde d'éthylène sur un ester préalablement séché, en présence d'un catalyseur basique, les conditions réactionnelles (quantité d'oxyde d'éthylène, pression, température et durée) étant déterminées en fonction du nombre total de molécules d'oxyde d'éthylène recherché.

Selon un deuxième aspect, la présente demande vise à couvrir les produits de traitement phytosanitaire ainsi que les médicaments à usage vétérinaire ou humain contenant une composition auto-émulsionnable essentiellement constituée d'au moins un composé de formule I, II ou III telle que définie précédemment.

Selon une caractéristique particulière, cette composition auto-émulsionnable peut en outre comprendre un solvant biodégradable, miscible avec lesdits esters d'acides gras éthoxylés, de préférence choisi parmi les triglycérides, les glycols, les esters et cétones légers.

Dans ce cas, la teneur en solvant biodégradable au sein de la composition auto-émulsionnable sera généralement inférieure ou égale à 50 % en poids.

D'une façon générale, des produits de traitement phytosanitaire ou pharmaceutique à usage vétérinaire ou humain comprendront au moins une matière active ou un principe actif en association avec une composition auto-émulsionnable dans des proportions relatives variant de environ 1/99 à 90/10 selon les principes actifs et l'application visée.

Selon un troisième aspect, la présente demande vise à couvrir un procédé de préparation d'un produit de traitement phytosanitaire ou pharmaceutique à usage vétérinaire ou humain se présentant sous forme d'émulsion stable, caractérisé en ce qu'il consiste à mélanger, pratiquement sans apport d'énergie, par exemple par dispersion par agitation mécanique lente, une phase aqueuse et une composition auto-émulsionnable comportant au moins un ester d'acide gras éthoxylé répondant à l'une des formules suivantes :

(I) 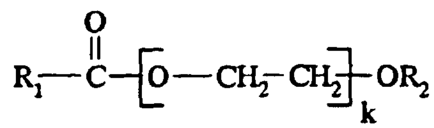

(II) 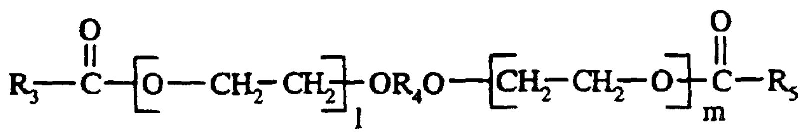

(III) 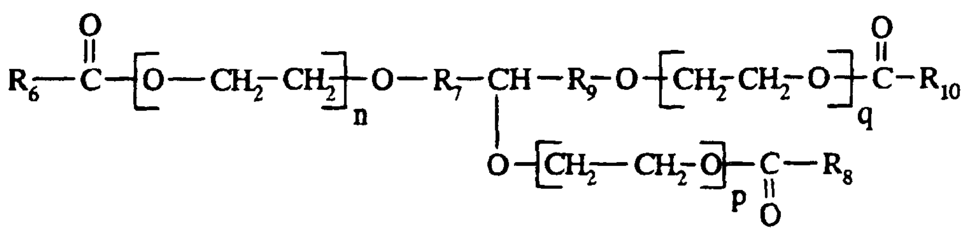

dans lesquelles :

- $R_1$, $R_3$, $R_5$, $R_6$, $R_8$ et $R_{10}$ représentent une chaîne hydrocarbonée, linéaire ou ramifiée, saturée ou insaturée ayant de 5 à 30 atomes de carbones ;
- $R_2$, $R_4$, $R_7$ et $R_9$ représentent une chaîne hydrocarbonée, linéaire ou ramifiée, saturée ou insaturée ayant de 1 à 5 atomes de carbone ;

le nombre total de molécules d'oxyde d'éthylène respectivement représenté dans les formules I, II et III précitées par k, l+m, n+p+q étant un nombre entier tel que la valeur HLB (balance hydrophile-lipophile) desdits composés soit comprise entre 4 et 10, de préférence entre 5 et 9.

D'une façon générale, la phase aqueuse et la composition auto-émulsionnable seront mélangées dans des proportions relatives variant d'environ 97/3 à environ 50/50, de préférence 95/5 à environ 70/30.

La phase aqueuse et la composition auto-émulsionnable précitées contiendront chacune de 0 à 100 % en poids du principe actif ou de la matière active caractérisant le produit.

L'invention sera illustrée plus en détail par les exemples suivants.

Dans ces exemples, les pourcentages sont exprimés en poids, sauf indication contraire.

**Exemple 1**

Préparation d'un ester méthylique d'acides gras oxyéthyléné.

On a préparé quatre produits (désignés ci-après A,B,C,D) par condensation d'oxyde d'éthylène sur des esters méthyliques obtenus à partir d'une coupe dérivée d'acides gras ayant 16 et 18 atomes de carbone et présentant la constitution suivante :

5 % Linolénate,
40% Linoléate
32 % Oléate,
5 % Ricinoléate,
6 % Stéarate,
6 % Palmitate,
QSP 100 % Autres

Ces esters méthyliques éthoxylés ont été préparés selon le procédé général suivant :

- chargement de l'ester méthylique dans un autoclave ;
- séchage sous vide à 120°C environ ;
- addition d'un catalyseur basique tel que potasse ou méthylate de sodium ;
- introduction de la quantité d'oxyde d'éthylène nécessaire à l'obtention du rapport molaire désiré, sous une pression de 4,5 bars ;
- maintien de la température à 180 °C environ pendant 45 minutes environ ;
- après refroidissement, neutralisation du catalyseur par un acide tel que l'acide formique ou l'acide acétique.

Les produits obtenus répondent à la formule I, dans laquelle $R_1$ représente une chaîne hydrocarbonée à 15 ou 17 atomes de carbone correspondant respectivement aux restes (par enlèvement d'un atome d'hydrogène) des acides palmitique, stéarique, ricinoléique, oléique, linoléique et linolénique, et $R_2$ représente un radical méthyl.

Les quatre produits ainsi obtenus ont été caractérisés par leur indice de saponification (IS) et par leur valeur HLB.

Le pouvoir auto-émulsionnant de ces produits a été évalué par une mesure de la stabilité d'émulsions préparées selon le protocole suivant :

- dans un bêcher de 150 ml, on pèse 20 g de l'ester éthoxylé étudié, puis on verse 80g d'eau de ville à température ambiante et on homogénéise le mélange ainsi obtenu par un tour de spatule.

Les résultats obtenus ont été reportés dans le tableau I ci-après, dans lequel on a également mentionné les quantités en poids d'huile et d'oxyde d'éthylène utilisées pour parvenir au dégré d'éthoxylation mentionné.

Tableau I

| PRODUIT | A | B | C | D |
|---|---|---|---|---|
| **Poids d'huile** | 100 | 100 | 100 | 100 |
| **Poids d'oxyde d'éthylène** | 15,4 | 31 | 46 | 77 |
| **Degré d'éthoxylation moyen (k)** | 1 | 2 | 3 | 5 |
| **ANALYSE:** | | | | |
| **- IS**<br>**- HLB**<br>**- Stabilité émulsion** | 167<br>3,1<br>< 1H | 147<br>5,2<br>+ de 4 jours | 127<br>7,2<br>2 jours | 102<br>9,7<br>QQ Heures |

On constate à la lecture de ce tableau que le produit B contenant 2 moles d'oxyde d'éthylène est le plus performant.

La valeur HLB de ce produit est de 5,2.

**Exemple 2**

Préparation d'une huile de colza éthoxylée

Cinq produits (désignés ci-après E,F,G,H,I) ont été préparés par condensation d'oxyde d'éthylène avec de l'huile de colza.

L'huile de colza utilisée dans cet exemple provient de colza français et comporte des chaînes grasses ayant de 16 à 22 atomes de carbone. Ces produits ont été obtenus par le procédé suivant :

- chargement dans un autoclave de 2200 g d'huile de colza , 44 g de glycérol (2%) et 7 g d'un catalyseur basique tel potasse ou méthylate de sodium ;
- séchage sous barbotage d'azote à 110°C pendant 10 minutes environ ;
- chauffage du mélange à 160-180°C puis introduction de la quantité d'oxyde d'éthylène nécessaire à l'obtention du rapport molaire désiré, sous une pression de 4,5 bars ;
- maintien de la température réactionnelle pendant 45 minutes environ;
- après refroidissement, neutralisation du catalyseur par un acide faible tel que l'acide formique ou l'acide acétique et filtration.

La présence de glycérol en un pourcentage relativement faible, c'est-à-dire inférieur à environ 5% en poids, et de préférence de l'ordre de 2% en poids exprimé par rapport à la charge en huile à éthoxyler facilite la réaction d'éthoxylation.

On obtient ainsi un produit de formule III dans laquelle :

$R_6$, $R_8$ et $R_{10}$ représentent des chaînes grasses ayant de 15 à 21 atomes de carbone correspondant aux chaînes grasses de l'huile de colza.

$$R_7 = R_9 = CH_2\text{-}$$

Les produits obtenus ont été caractérisés et évalués en utilisant les mêmes protocoles expérimentaux que ceux mentionnés à l'exemple 1 et les résultats obtenus ont été réportés dans le tableau II ci-après.

Tableau II

| **PRODUIT** | **E** | **F** | **G** | **H** | **I** |
|---|---|---|---|---|---|
| **Poids d'huile** | 2,2 | 2,2 | 2,2 | 2,2 | 2,2 |
| **Poids d'oxyde d'éthylène** | 0,33 | 0,65 | 1,1 | 2,2 | 3,3 |
| **degré d'éthoxylation moyen (n + p + q)** | 3 | 6 | 10 | 20 | 30 |
| **Analyse: I.S** | 159 | 141 | 120 | 90 | 69 |
| **HLB** | 2,1 | 4,1 | 6,5 | 9,9 | 12,2 |
| **Stabilité d'émulsion** | < 10mn | < 10mn | 10 mn | 24 H | 10 mn |

On constate que le triester de glycérol éthoxylé H (comportant 20 moles d'oxyde d'éthylène) est le plus performant.

**Exemple 3**

Préparation d'une composition auto-émulsionnable contenant un ester méthylique d'acide gras et un solvant biodégradable.

Le produit B décrit à l'exemple 1 (comportant 2 moles d'oxyde d'éthylène) présente une densité de 0,94 et une viscosité de 28 mPas à 25°C.

Ce produit présente une bonne dispersibilité c'est-à-dire une bonne aptitude à se mélanger avec de l'eau sans agitation.

Il a été observé que cette dispersibilité dans l'eau peut être encore améliorée en mélangeant ce produit à

une quantité équivalente environ d'un solvant biodégradable, de préférence de densité supérieure à 1 pour obtenir une composition de densité voisine de 1.

Des solvants pouvant être utilisés à cet effet sont par exemple le propylèneglycol, la glycérine, le triacétate de glycérol.

Le tableau III ci-après mentionne les propriétés de dispersibilité et de stabilité d'émulsions obtenues selon le même mode opératoire, avec le produit B de l'exemple I et avec un mélange 50/50 de ce produit avec du triacétate de glycérol.

Tableau III

| COMPOSITION AUTO-EMULSIONNABLE | PRODUIT B SEUL | PRODUIT B (50%) TRIACETATE DE GLYCEROL (50 %) |
|---|---|---|
| DISPERSIBILITE | BONNE | TRES BONNE |
| STABILITE EMULSION | + DE 4 JOURS | 4 JOURS |

**Exemple 4**

Préparation d'une composition auto-émulsionnable contenant un mélange de mono et de triester éthoxylé

Le triester de glycérol éthoxylé H préparé à l'exemple 2 (comportant 20 moles d'oxyde d'éthylène) présente une densité de 1,01 et une viscosité de 170 mPas à 25°C.

Les caractéristiques de densité et de viscosité du produit B de l'exemple 1 ont été mentionnées à l'exemple 3.

Les produits B et H précités ont été mélangés dans des proportions variables pour obtenir des compositions auto-émulsionnables de densité voisine de 1 et de faible viscosité.

Le tableau IV ci-après résume les principales caractéristiques des produits B et H ainsi que de leurs mélanges.

Tableau IV

| | | | MELANGES PRODUIT B / PRODUIT H | | |
|---|---|---|---|---|---|
| Composition | Produit B | Produit H | 20/80 | 40/60 | 80/20 |
| Viscosité | 28 mPas | 170 mPas | 113 mPas | 85 mPas | 30 mPas |
| Densité | 0,94 | 1,01 | 0,99 | 0,98 | 0,96 |
| Dispersibilité | Bonne | Moyenne | Assez bonne | Bonne | Bonne |
| Stabilité émulsion | > 4 Jours | 1 Jour | 1 Jour | 2 Jours | 4 Jours |

Les mélanges des produits B et H, en particulier le mélange 80/20 constituent des phases auto-émulsionables très intéressantes.

**Exemple 5**

Préparation de compositions auto-émulsionnables contenant des huiles de ricin éthoxylées

De l'oxyde d'éthylène est condensée en différentes proportions avec des huiles de ricin première pression.

Le procédé de synthèse mis en oeuvre est semblable à celui généralement décrit à l'exemple 2, si ce n'est qu'il est réalisé en l'absence de glycérol.

Deux produits (désignés ci-après J et K) ont été ainsi préparés.

On a également préparé une composition contenant le produit J en mélange avec du propylène glycol dans des proportions relatives de 75/25.

Le pouvoir auto-émulsionnant de ces trois produits a été évalué selon le protocole décrit à l'exemple 1, si ce n'est que le mélange avec l'eau a été réalisé par une agitation manuelle de 6 battements du flacon de 100 ml, pré-

lablement bouché.

Les résultats obtenus sont mentionnés au Tableau V ci-après.

Tableau V

| PRODUIT | J | K | PRODUIT J 75 % PROPYLENE GLYCOL 25 % |
|---|---|---|---|
| **Poids d'huile** | 4 | 4 | - |
| **Poids d'oxyde d'éthylène** | 1 | 1,5 | - |
| **Nombre moyen de Moles OE (n+p+q)** | 5 | 7 | - |
| **IS** | 143 | 130 | - |
| **HLB** | 4,8 | 5,4 | 4,8 |
| **Stabilité émulsion** | 3 Jours | 5 Heures | 12 Heures |
| **Dispersibilité** | Moyenne | Moyenne | Bonne |

**Exemple 6**

Préparation d'esters méthyliques de colza éthoxylés

Un ester méthylique d'huile de colza ayant les caractéristiques suivantes :

| | |
|---|---|
| Aspect | Limpide |
| Indice d'acide | 0.4 |
| Indice de réfraction à 20°C | 1.4562 |
| Couleur Gardner | 3- |
| Indice de Saponification | 189 |
| Teneur en ester méthylique | 96.5 % |

a été éthoxylé par 2,4,6 ou 8 moles d'oxyde d'éthylène.

Le procédé de synthèse utilisé est celui décrit à l'exemple 1.

Les produits ainsi obtenus (désignés respectivement L, M, N et O) correspondent à des mélanges de produits répondant à la formule générale I, dans laquelle $R_1$ représente une chaîne hydrocarbonnée de 15 à 19 atomes de carbone correspondant aux chaînes grasses de l'huile de colza et $R_2$ représente un groupement $CH_3$.

Ces produits sont mis en émulsion en utilisant le protocole décrit à l'exemple 1.

Le tableau VI ci-après mentionne les propriétés des quatre produits synthétisés et de leurs émulsions.

Tableau VI

| PRODUIT | L | M | N | O |
|---|---|---|---|---|
| **Poids d'huile** | 3,7 | 3,7 | 3,7 | 3,7 |
| **Poids d'oxyde d'éthylène** | 1,1 | 2,2 | 3,3 | 4,4 |
| **Nombre OE (k)** | 2 | 4 | 6 | 8 |
| **IS** | 141 | 116 | 89 | 73 |
| **HLB** | 5,9 | 8,4 | 11,1 | 12,7 |
| **Dispersibilité dans l'eau** | Très bonne | Très bonne | Bonne | Moyenne |
| **Stabilité émulsion** | > 2 heures | > 2 Heures | 2 Heures | < 15 Minutes |

Les produits L et M (comportant respectivement 2 et 4 moles d'oxyde d'éthylène) s'avèrent les plus performants.

**Exemple 7**

Comparaison des compositions auto-émulsionnables selon l'invention à des compositions commerciales

On a comparé les compositions auto-émulsionnables conformes à la présente invention, à deux huiles commerciales destinées à la préparation de concentrés émulsionnables de produits phytosanitaires.
Les caractéristiques de ces huiles commerciales sont les suivantes :

- Huile 11E : Huile minérale de type paraffinique contenant des agents tensioactifs de type alkyl-phénols ayant obtenue l'autorisation de commercialisation n° 6700013 comme adjuvant pour herbicides ;
- AGRIROB CM® : Huile végétale contenant des agents tensioactifs, ayant obtenue l'autorisation de commercialisation 8600162 comme adjuvant pour bouillie herbicide.

Ces deux huiles commerciales ont été comparées au produit B de l'exemple 1, M de l'exemple 6 et H de l'exemple 2.
Plus précisément, on a mélangé à chacun de ces produits, différents actifs phytosanitaires (ETHEPHON®, Chlorpropham®, Formol-Bromure de lauryl ammonium) à des concentrations habituellement utilisées.
La dispersion dans l'eau des huiles a été réalisée en suivant le protocole expérimental décrit à l'exemple 1.
Les résultats obtenus ont été reportés dans les tableaux VII à X ci-après.
Dans chaque cas, on a mesuré la stabilité du mélange (huile-actif phytosanitaire), la dispersibilité, ainsi que la stabilité de la dispersion aqueuse.

Tableau VII

| **Huiles sans actif** | | | | | |
|---|---|---|---|---|---|
| **Huiles** | **11E** | **Agrirob CM ®** | **Produit B** | **Produit M** | **Produit H** |
| **Aspect huile** | limpide stable | limpide stable | jaune, stable | jaune, stable | jaune ; stable |
| **Dispersibilité** | Bonne | Très bonne | Bonne | Bonne | Assez Bonne |
| **Stabilité émulsion** | #2H | #2H | >=6H | >=6H | <2H |

Tableau VIII

| Formulations avec modulateur de croissance (ETHEPHON) | | | | | |
|---|---|---|---|---|---|
| Ethepton 120 g<br>Huile 880 g | | | | | |
| **Huiles** | **11E** | **Agrirob CM ®** | **Produit B** | **Produit M** | **Produit H** |
| **Aspect ; stabilité formulation** | Instable | Instable | Stable; Limpide | Stable ; Limpide | Stable Limpide |
| **Dispersibilité** | Moyenne | Moyenne | Très bonne | Bonne | Moyenne |
| **Stabilité émulsion 25*** | < 15 mn | < 15 mn | > 24 H | > 24 H | < 15 mn |
| **Stabilité émulsion 40*** | Instable | Instable | > 24 H | > 24 H | Instable |

Tableau IX

| Formulations avec herbicide (Chlorpropham) | | | | |
|---|---|---|---|---|
| Chlorpropham 450 g<br>Huile 550 g | | | | |
| **Huiles** | **11E** | **Agricob CM ®** | **Produit B** | **Produit H** |
| **Aspect; stabilité formulation** | Instable | Limpide Stable | Limpide Stable | Limpide Stable |
| **Dispersibilité** | Moyenne | Moyenne | Moyenne | Moyenne |

Tableau IX (suite)

| Formulations avec herbicide (Chlorpropham) | | | | |
|---|---|---|---|---|
| **Huiles** | **11E** | **Agricob CM ®** | **Produit B** | **Produit H** |
| **Stabilité émulsion 25°C** | < 15 mn | < 2 H | < 15 mn | # 24 H |
| **Stabilité émulsion 40°C** | < 15 mn | Instable | Instable | > 24 H |

Tableau X

| Formulations biocides | | | | | |
|---|---|---|---|---|---|
| Formol 30 % 25 g<br>Bromure de lauryl ammonium 25 g<br>Huile 950 g | | | | | |
| **Huiles** | **11E** | **Agrirob CM®** | **Produit B** | **Produit M** | **Produit H** |
| **Aspect ; stabilité formulation** | Instable | Instable | Limpide Stable | Voilé Stable 24 H | Limpide Stable |
| **Dispersibilité** | Moyenne | Moyenne | Bonne | Bonne | Moyenne |
| **Stabilité émulsion 25°C** | < 15 mn | # 2 H | > 24 H | > 24 H | > 24 H |
| **Stabilité émulsion 40°C** | < 15 mn | Instable | > 24 H | > 24 H | > 24 H |

Les résultats mentionnés dans les tableaux qui précèdent, démontrent que les compositions auto-émulsionnables conformes à la présente invention permettent d'obtenir des formulations de principes actifs phytosanitaires au moins aussi stables, et souvent plus stables, que les huiles commerciales actuellement disponibles.

## Exemple 8

Utilisation des compositions auto-émulsionnables selon l'invention pour la préparation de médicaments

L'utilisation d'émulsions est préconisée dans le domaine pharmaceutique notamment dans le cas où l'on met en oeuvre un principe actif liposoluble, ou encore dans le cas où l'on recherche un effet retard.

Les huiles auto-émulsionnables sont particulièrement utiles pour la préparation d'émulsions de façon extemporanée, par simple agitation manuelle ou au moyen de dispositifs simples tels que des seringues, dans le cas de préparations injectables.

Les huiles auto-émulsionnables susceptibles d'être utilisées pour de telles applications doivent bien entendu être dépouvues de tout effet toxique.

Un exemple particulier de produits pharmaceutiques dans lequel des huiles auto-émulsionnables peuvent être utilisées est celui des vaccins huileux injectables.

Les produits B de l'exemple 1 et H de l'exemple 2 ont été utilisés pour la préparation de deux vaccins contenant de l'albumine sérique bovine comme antigène modèle.

Des doses de 100 μl de vaccins ont été injectées à des souris de type OF1 femelles par voie sous-cutanée.

Chaque dose contenait 50 μg d'albumine et 25 μl d'huile. A titre de témoin, une préparation contenant 50 μg d'albumine et 25 μl d'un adjuvant huileux commercial a été injectée à un troisième groupe de souris.

Cet adjuvant huileux commercial est le produit Montanide® ISA25 (huile minérale, oléate de mannitol).

Un quatrième groupe d'animaux a reçu une solution aqueuse d'albumine sans huile.

Le dosage des anticorps anti-albumine (IgG totales) est réalisé par une technique EUSA classique, 42 jours après injection.

Les résultats obtenus ont été reportés dans le tableau XI ci-après.

Tableau XI

| **Groupe** | **1** | **2** | **3** | **4** |
|---|---|---|---|---|
| **Huile** | Produit B | Produit H | Montanide ISA® | Sans huile |

Tableau XI (suite)

| Groupe | 1 | 2 | 3 | 4 |
|---|---|---|---|---|
| **Taux anticorps** | 20000 | 20000 | 20000 | 0 |

Ce tableau montre que les deux huiles auto-émulsionnables conformes à la présente invention permettent d'accroître la réponse immunitaire à 42 jours par rapport au groupe témoin n° 4 (solution aqueuse d'albumine sans huile).

Le niveau de cette réponse immunitaire est équivalent à celui obtenu avec l'adjuvant commercial.

Aucune réaction d'intolérance n'est observée chez les animaux, ni aux sites d'injection, ni au niveau de leur comportement général.

Les huiles conformes à la présente invention peuvent donc être utilisées pour fabriquer des émulsions contenant un principe actif pharmaceutique.

A titre d'exemple, un tel principe actif peut être un antibiotique, un antigène, un anti-inflammatoire, un anti-asthmatique, etc.

## Revendications

**1.** Utilisation d'esters d'acides gras éthoxylés répondant à l'une des formules suivantes :

(I) 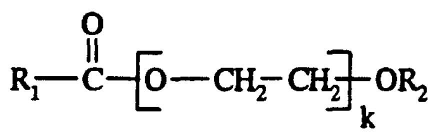

(II) 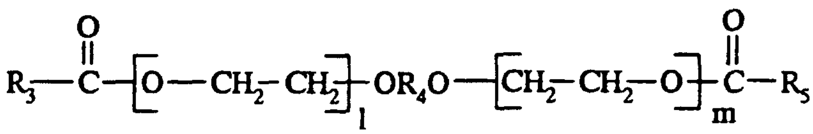

(III) 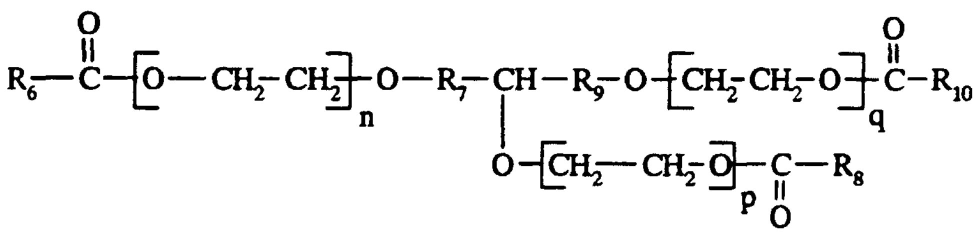

dans lesquelles :

- $R_1$, $R_3$, $R_5$, $R_6$, $R_8$ et $R_{10}$ représentent une chaîne hydrocarbonée, linéaire ou ramifiée, saturée ou insaturée ayant de 5 à 30 atomes de carbones ;
- $R_2$, $R_4$, $R_7$ et $R_9$ représentent une chaîne hydrocarbonée, linéaire ou ramifiée, saturée ou insaturée ayant de 1 à 5 atomes de carbone ;

le nombre total de molécules d'oxyde d'éthylène respectivement représenté dans les formules I, II et III précitées par k, l+m, n+p+q étant un nombre entier tel que la valeur HLB (balance hydrophile-lipophile) desdits composés soit comprise entre 4 et 10, de préférence entre 5 et 9 ;

comme composants auto-émulsionnables notamment utiles pour la préparation de produits phytosanitaires ou de médicaments à usage vétérinaire ou humain.

**2.** Utilisation selon la revendication 1 d'esters d'acides gras éthoxylés répondant à la formule I précitée dans laquelle $R_1$ est choisi parmi les restes des acides palmitique, stéarique, ricinoléique, oléique, linoléique et linolénique ; $R_2$ représente un radical méthyl et k est un nombre entier compris entre 1 et 5, de préférence égal à 2.

**3.** Utilisation selon la revendication 1 d'esters d'acides gras éthoxylés répondant à la formule III précitée dans laquelle :

- $R_6$, $R_8$ et $R_{10}$ représentent des chaînes hydrocarbonées correspondant aux chaînes grasses d'une huile végétale ;
- $R_7$ et $R_9$ représentent un groupe méthylène $CH_2$ ;
- n, p, q présentent des nombres entiers tels que leur somme soit comprise entre 3 et 30.

**4.** Utilisation selon la revendication 1 d'esters d'acides gras éthoxylés répondant à la formule III précitée dans laquelle :

- $R_6$, $R_8$ et $R_{10}$ représentent des chaînes hydrocarbonées correspondant aux chaînes grasses de l'huile de colza ;
- $R_7$ et $R_9$ représentent un groupe méthylène $CH_2$ ;
- n, p, q présentent des nombres entiers tels que leur somme soit comprise entre 3 et 30, et de préférence égale à 20.

**5.** Utilisation selon la revendication 1 d'esters d'acides gras éthoxylés répondant à la formule III précitée dans laquelle :

- $R_6$, $R_8$ et $R_{10}$ représentent des chaînes hydrocarbonées correspondant aux chaînes grasses de l'huile de ricin ;
- $R_7$ et $R_9$ représentent un radical méthylène $CH_2$ ;
- n, p et q représentent des nombres entiers tels que leur somme soit comprise entre 5 et 7.

**6.** Produit de traitement phytosanitaire ou pharmaceutique à usage vétérinaire ou humain, **caractérisé en ce qu'**il comprend au moins une matière active ou un principe actif en association avec une composition auto-émulsionnable comportant au moins un ester d'acide gras éthoxylé répondant à l'une des formules suivantes :

(I) 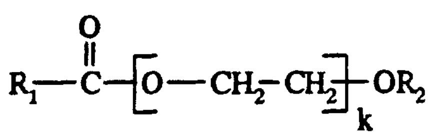

(II) 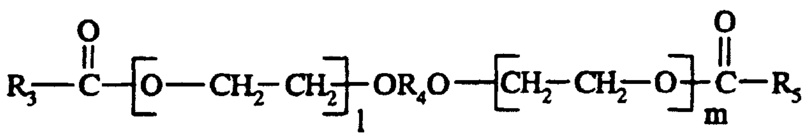

(III) 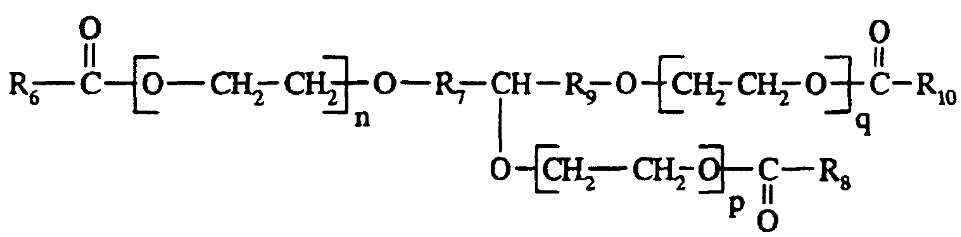

dans lesquelles :

- $R_1$, $R_3$, $R_5$, $R_6$, $R_8$ et $R_{10}$ représentent une chaîne hydrocarbonée, linéaire ou ramifiée, saturée ou insaturée ayant de 5 à 30 atomes de carbones ;
- $R_2$, $R_4$, $R_7$ et $R_9$ représentent une chaîne hydrocarbonée, linéaire ou ramifiée, saturée ou insaturée ayant de 1 à 5 atomes de carbone ;

le nombre total de molécules d'oxyde d'éthylène respectivement représenté dans les formules I, II et III précitées par k, l+m, n+p+q étant un nombre entier tel que la valeur HLB (balance hydrophile-lipophile) desdits composés soit comprise entre 4 et 10, de préférence entre 5 et 9.

**7.** Produit selon la revendication 6, **caractérisé en ce que** la composition auto-émulsionnable précitée contient au moins un ester d'acide gras éthoxylé répondant à la formule I précitée dans laquelle $R_1$ est choisi parmi les restes des acides palmitique, stéarique, ricinoléique, oléique, linoléique et linolénique ; $R_2$ représente un radical méthyl et k est un nombre entier compris entre 1 et 5, de préférence égal à 2.

**8.** Produit selon la revendication 6 ou 7, **caractérisé en ce que** la composition auto-émulsionnable précitée comprend au moins un ester d'acide gras éthoxylé répondant à la formule III précitée dans laquelle :

- $R_6$, $R_8$ et $R_{10}$ représentent des chaînes hydrocarbonées correspondant aux chaînes grasses d'une huile végétale ;
- $R_7$ et $R_9$ représentent un groupe méthylène $CH_2$;
- n, p, q présentent des nombres entiers tels que leur somme soit comprise entre 3 et 30.

**9.** Produit selon la revendication 6 ou 7, **caractérisé en ce que** la composition auto-émulsionnable précitée comprend au moins un ester d'acide gras éthoxylé répondant à la formule III précitée dans laquelle :

- $R_6$, $R_8$ et $R_{10}$ représentent des chaînes hydrocarbonées correspondant aux chaînes grasses de l'huile de colza ;
- $R_7$ et $R_9$ représentent un groupe méthylène $CH_2$ ;
- n, p, q présentent des nombres entiers tels que leur somme soit comprise entre 3 et 30, et de préférence égale à 20.

**10.** Produit selon la revendication 6 ou 7, **caractérisé en ce que** la composition auto-émulsionnable précitée comprend au moins un ester d'acide gras éthoxylé répondant à la formule III précitée dans laquelle :

- $R_6$, $R_8$ et $R_{10}$ représentent des chaînes hydrocarbonées correspondant aux chaînes grasses de l'huile de ricin ;
- $R_7$ et $R_9$ représentent un radical méthylène $CH_2$ ;
- n, p et q représentent des nombres entiers tels que leur somme soit comprise entre 5 et 7.

**11.** Produit selon l'une des revendications 6 à 10, **caractérisé en ce que** la composition auto-émulsionnable précitée comprend en outre un solvant biodégradable, miscible avec lesdits esters d'acides gras éthoxylés, de préférence choisi parmi les triglycérides, les glycols, les esters ou cétones légers

**12.** Produit selon la revendication 11, **caractérisé en ce que** la composition auto-émulsionnable précitée est constituée de :

50 à 100 % en poids d'un ou plusieurs esters d'acides gras éthoxylés tels que définis à l'une des revendications 5 à 8; et
0 à 50 % en poids d'un solvant biodégradable tel que défini à la revendication 9.

**13.** Produit selon l'une des revendications 6 à 12, **caractérisé en ce qu'**il comprend au moins une matière active ou un principe actif en association avec une composition auto-émulsionnable dans des proportions relatives variant de 1/99 à 90/10.

**14.** Produit selon l'une des revendications 6 à 13, **caractérisé en ce qu'**il comporte en outre une phase aqueuse et se présente sous forme d'émulsion stable.

**15.** Procédé de préparation d'un produit de traitement phytosanitaire ou pharmaceutique à usage vétérinaire ou humain se présentant sous forme d'émulsion stable, **caractérisé en ce qu'**il consiste à mélanger, pratiquement sans

apport d'énergie, par exemple par dispersion par agitation mécanique lente, une phase aqueuse, et une composition auto-émulsionnable comportant au moins un ester d'acide gras éthoxylé répondant à l'une des formules suivantes :

(I) 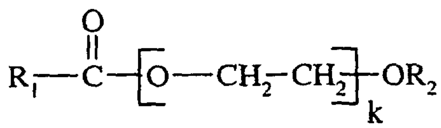

(II) 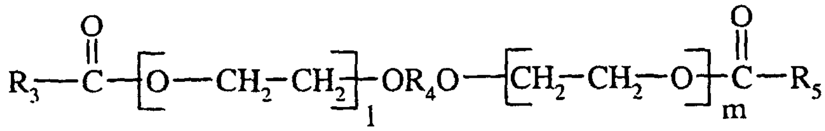

(III) 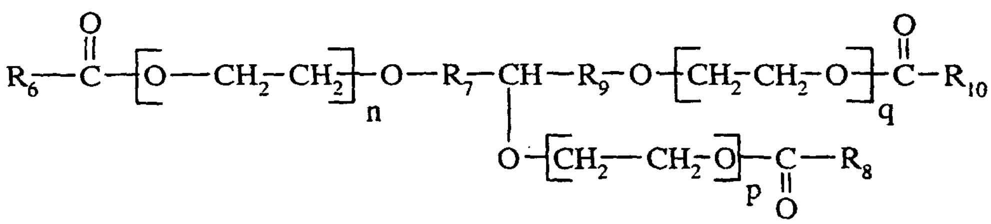

dans lesquelles :

- $R_1$, $R_3$, $R_5$, $R_6$, $R_8$ et $R_{10}$ représentent une chaîne hydrocarbonée, linéaire ou ramifiée, saturée ou insaturée ayant de 5 à 30 atomes de carbones ;
- $R_2$, $R_4$, $R_7$ et $R_9$ représentent une chaîne hydrocarbonée, linéaire ou ramifiée, saturée ou insaturée ayant de 1 à 5 atomes de carbone ;

le nombre total de molécules d'oxyde d'éthylène respectivement représenté dans les formules I, II et III précitées par k, l+m, n+p+q étant un nombre entier tel que la valeur HLB (balance hydrophile-lipophile) desdits composés soit comprise entre 4 et 10, de préférence entre 5 et 9.

## Claims

**1.** Use of ethoxylated fatty acid esters having one of the following formulae :

(I) 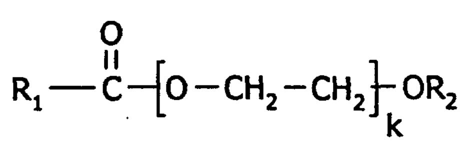

(II) 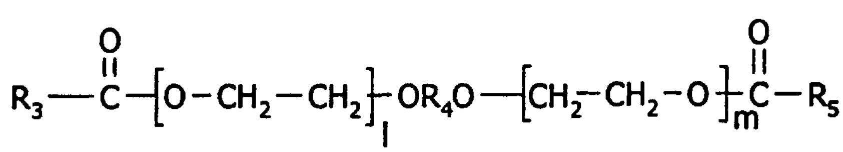

(III) 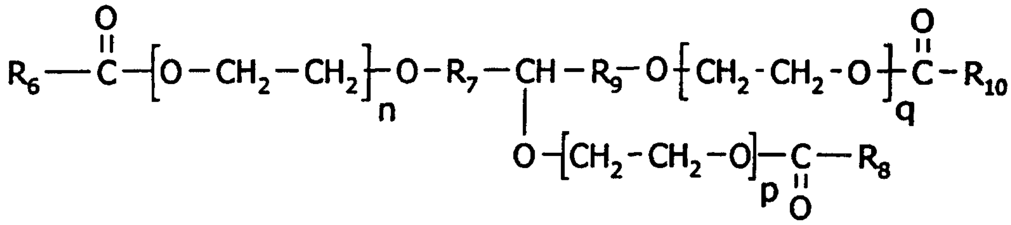

in which

- $R_1$, $R_3$, $R_5$, $R_6$, $R_8$ and $R_{10}$ represent a linear or branched, saturated or unsaturated hydrocarbon chain having 5 to 30 carbon atoms;
- $R_2$, $R_4$, $R_7$ and $R_9$ represent a linear or branched, saturated or unsaturated hydrocarbon chain having 1 to 5 carbon atoms ;

the total number of ethylene oxide molecules represented in the above-mentioned formulae I, II and III by k, l+m, n+p+q respectively being an integer such that the HLB value (hydrophilic-lipophilic balance) of said compounds be between 4 and 10, preferably between 5 and 9;
as self-emulsifiable components, useful notably for preparing phytosanitary products or medicaments for veterinary or human use.

2. The use according to claim 1 of ethoxylated fatty acid esters having the above-mentioned formula I in which $R_1$ is selected from palmitic, stearic, ricinoleic, oleic, linoleic and linolenic acid residues ; $R_2$ represents a methyl radical and k is an integer between 1 and 5, preferably equal to 2.

3. The use according to claim 1 of ethoxylated fatty acid esters having the above-mentioned formula III in which :

- $R_6$, $R_8$ and $R_{10}$ represent hydrocarbon chains corresponding to the fatty chains of a vegetable oil;
- $R_7$ and $R_9$ represent a methylene group $CH_2$ ; and
- n, p, q are integers such that their sum be between 3 and 30.

4. The use according to claim 1 of ethoxylated fatty acid esters having the above-mentioned formula III in which :

- $R_6$, $R_8$ and $R_{10}$ represent hydrocarbon chains corresponding to the fatty chains of rapeseed oil;
- $R_7$ and $R_9$ represent a methylene group $CH_2$ ; and
- n, p, q are integers such that their sum be between 3 and 30, and preferably equal to 20.

5. The use according to claim 1 of ethoxylated fatty acid esters having the above-mentioned formula III in which :

- $R_6$, $R_8$ and $R_{10}$ represent hydrocarbon chains corresponding to the fatty chains of castor oil;
- $R_7$ and $R_9$ represent a methylene radical $CH_2$ ; and
- n, p, q represent integers such that their sum be between 5 and 7.

6. A phytosanitary or pharmaceutical treatment product for veterinary or human use, **characterised in that** it comprises at least one active material or at least one active principle in combination with a self-emulsifiable composition having at least one ethoxylated fatty acid ester having one of the following formulae :

(I) 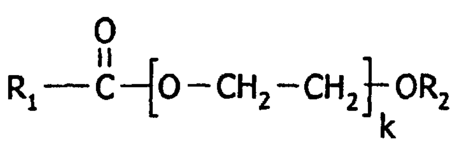

(II) 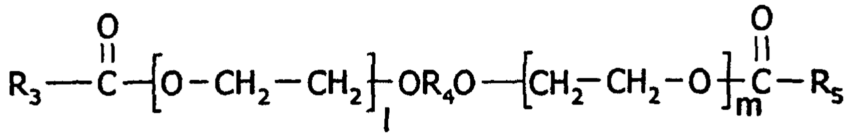

(III) 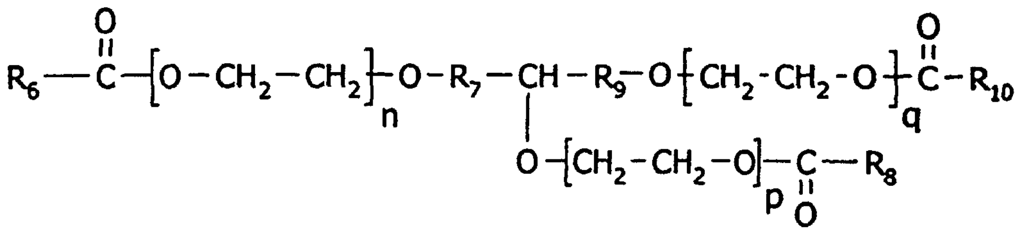

in which :

- $R_1$, $R_3$, $R_5$, $R_6$, $R_8$ and $R_{10}$ represent a linear or branched, saturated or unsaturated hydrocarbon chain having 5 to 30 carbon atoms;
- $R_2$, $R_4$, $R_7$ and $R_9$ represent a linear or branched, saturated or unsaturated hydrocarbon chain having 1 to 5 carbon atoms; the total number of ethylene oxide molecules represented in the above-mentioned formulae I, II and III by k, l+m, n+p+q respectively being an integer such that the HLB value (hydrophilic-lipophilic balance) of said compounds be between 4 and 10, preferably between 5 and 9.

**7.** The product according to claim 6, **characterised in that** the above-mentioned self-emulsifiable composition contains at least one ethoxylated fatty acid ester having the above-mentioned formula I in which $R_1$ is selected from palmitic, stearic, ricinoleic, oleic, linoleic and linolenic acid residues ; $R_2$ represents a methyl radical and k is an integer between 1 and 5, preferably equal to 2.

**8.** The product according to claim 6 or 7, **characterised in that** the above-mentioned self-emulsifiable composition comprises at least one ethoxylated fatty acid ester having the above-mentioned formula III in which :

- $R_6$, $R_8$ and $R_{10}$ represent hydrocarbon chains corresponding to the fatty chains of a vegetable oil;
- $R_7$ and $R_9$ represent a methylene group $CH_2$; and
- n, p, q are integers such that their sum be between 3 and 30.

**9.** The product according to claim 6 or 7, **characterised in that** the above-mentioned self-emulsifiable composition comprises at least one ethoxylated fatty acid ester having the above-mentioned formula III in which :

- $R_6$, $R_8$ and $R_{10}$ represent hydrocarbon chains corresponding to the fatty chains of rapeseed oil ;
- $R_7$ and $R_9$ represent a methylene group $CH_2$; and
- n, p, q are integers such that their sum be between 3 and 30, and preferably equal to 20.

**10.** The product according to claim 6 or 7, **characterised in that** the above-mentioned self-emulsifiable composition comprises at least one ethoxylated fatty acid ester having the above-mentioned formula III in which :

- $R_6$, $R_8$ and $R_{10}$ represent hydrocarbon chains corresponding to the fatty chains of castor oil;
- $R_7$ and $R_9$ represent a methylene radical $CH_2$; and
- n, p, q are integers such that their sum be between 5 and 7.

**11.** The product according to one of claims 6 to 10, **characterised in that** the above-mentioned self-emulsifiable composition further comprises a biodegradable solvent which is miscible with said ethoxylated fatty acid esters preferably selected from triglycerides, glycols, low-molecular weight esters or ketones.

**12.** The product according to claim 11, **characterised in that** the above-mentioned self-emulsifiable composition is constituted of:

50 to 100 % by weight of one or more ethoxylated fatty acid esters as defined in one of claims 5 to 8; and
0 to 50 % by weight of a biodegradable solvent as defined in claim 9.

**13.** The product according to one of claims 6 to 12, **characterised in that** it comprises at least one active material or at least one active principle in combination with a self-emulsifiable composition in relative proportions ranging from 1/99 to 90/10.

**14.** The product according to one of claims 6 to 13, **characterised in that** it further contains an aqueous phase and is in the form of a stable emulsion.

**15.** A method of preparing a phytosanitary or pharmaceutical treatment product, in the form of a stable emulsion, for veterinary or human use, **characterised in that** it consists in mixing, practically without bringing about energy, by dispersion by slow mechanical stirring for example, an aqueous phase, and a self-emulsifiable composition having at least one ethoxylated fatty acid ester having one of the following formulae :

(I) 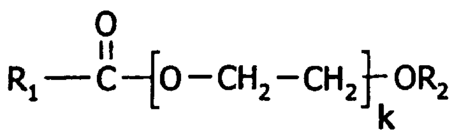

(II) 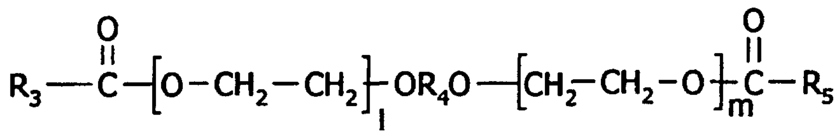

(III) 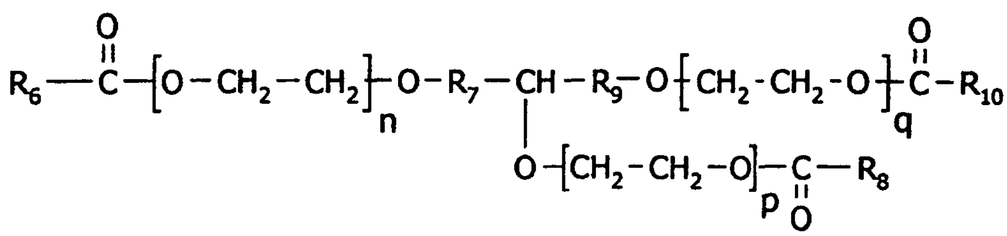

in which:

- $R_1$, $R_3$, $R_5$, $R_6$, $R_8$ and $R_{10}$ represent a linear or branched, saturated or unsaturated hydrocarbon chain having from 5 to 30 carbon atoms;
- $R_2$, $R_4$, $R_7$ and $R_9$ represent a linear or branched, saturated or unsaturated hydrocarbon chain having from 1 to 5 carbon atoms ;

the total number of ethylene oxide molecules represented in the above-mentioned formulae I, II and III by k, l+m, n+p+q respectively being an integer such that the HLB value (hydrophilic-lipophilic balance) of said compounds be between 4 and 10, preferably between 5 and 9.

## Patentansprüche

**1.** Verwendung von ethoxylierten Fettsäureestern, die einer der folgenden Formeln entsprechen:

(I) 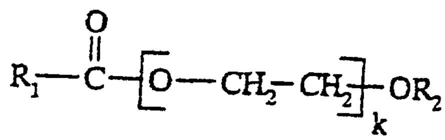

(II) 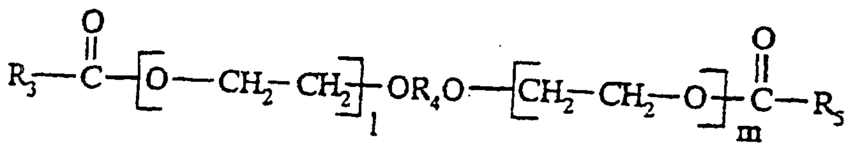

(III) 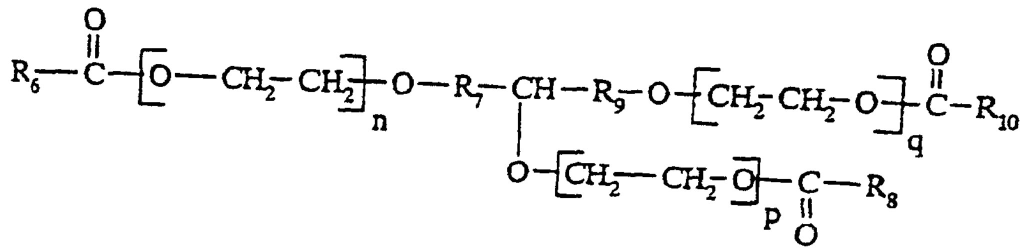

wobei:

- $R_1$, $R_3$, $R_5$, $R_6$, $R_8$ und $R_{10}$ eine lineare oder verzweigte, gesättigte oder ungesättigte Kohlenwasserstoffkette mit 5 bis 30 Kohlenstoffatomen darstellen;
- $R_2$, $R_4$, $R_7$ und $R_9$ eine lineare oder verzweigte, gesättigte oder ungesättigte Kohlenwasserstoffkette mit 1 bis 5 Kohlenstoffatomen darstellen;

die Gesamtzahl der Ethylenoxidmoleküle, die jeweils in den vorstehenden Formeln I, II und III durch k, l+m, n+p+q darstellt wird, eine ganze Zahl ist, so dass der HLB-Wert (hydrophiles-lipophiles Gleichgewicht) der Verbindungen zwischen 4 und 10, vorzugsweise zwischen 5 und 9 liegt;

als selbst-emulgierbare Komponenten, die besonders nützlich sind für die Herstellung von phytosanitären Produkten oder von Medikamenten zur Verwendung in der Veterinär- oder Humanmedizin.

**2.** Verwendung von ethoxylierten Fettsäureestern nach Anspruch 1, die der vorstehenden Formel I entsprechen, in der $R_1$ ausgewählt ist aus den Resten der Palmitinsäure, Stearinsäure, Ricinolsäure, Ölsäure, Linolsäure und Linolensäure; $R_2$ eine Methylgruppe darstellt und k eine ganze Zahl zwischen 1 und 5, vorzugsweise gleich 2 ist.

**3.** Verwendung von ethoxylierten Fettsäureestern nach Anspruch 1, die der vorstehenden Formel III entsprechen, in der:

- $R_6$, $R_8$ und $R_{10}$ Kohlenwasserstoffketten darstellen, die den Fettsäureketten eines Pflanzenöls entsprechen;
- $R_7$ und $R_9$ eine Methylengruppe $CH_2$darstellen;
- n, p und q ganze Zahlen darstellen, so dass ihre Summe zwischen 3 und 30 liegt.

**4.** Verwendung von ethoxylierten Fettsäureestern nach Anspruch 1, die der vorstehenden Formel III entsprechen, in der:

- $R_6$, $R_8$ und $R_{10}$ Kohlenwasserstoffketten darstellen, die den Fettsäureketten von Rapsöl entsprechen;
- $R_7$ und $R_9$ eine Methylengruppe $CH_2$darstellen;
- n, p und q ganze Zahlen darstellen, so dass ihre Summe zwischen 3 und 30 liegt und bevorzugt gleich 20 ist.

**5.** Verwendung von ethoxylierten Fettsäureestern nach Anspruch 1, die der vorstehenden Formel III entsprechen, in der:

- $R_6$, $R_8$ und $R_{10}$ Kohlenwasserstoffketten darstellen, die den Fettsäureketten von Rizinusöl entsprechen;
- $R_7$ und $R_9$ eine Methylengruppe $CH_2$darstellen;
- n, p und q ganze Zahlen darstellen, so dass ihre Summe zwischen 5 und 7 liegt.

**6.** Produkt zur phytosanitären Behandlung oder zur pharmazeutischen Behandlung zur Verwendung in der Veterinär- oder Humanmedizin, **dadurch gekennzeichnet, dass** es mindestens ein aktives Material oder einen aktiven Stoff in Verbindung mit einer selbst-emulgierbaren Zusammensetzung umfasst, die mindestens einen ethoxylierten Fettsäureester umfasst, der einer der folgenden Formeln entspricht:

(I) 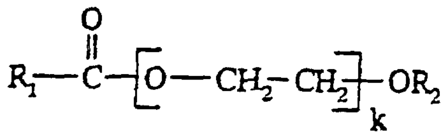

(II) 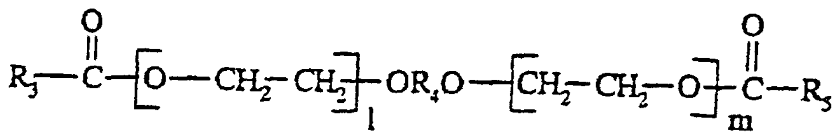

(III) 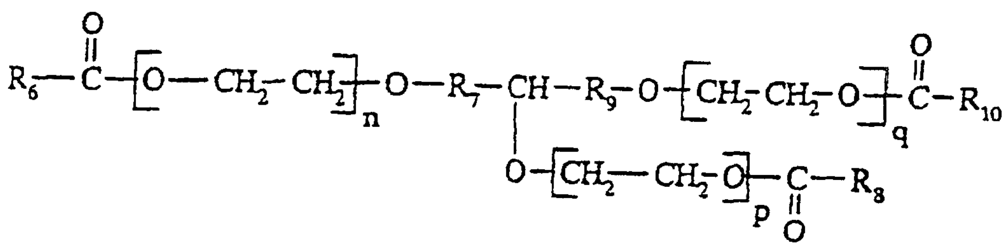

wobei:

- $R_1$, $R_3$, $R_5$, $R_6$, $R_8$ und $R_{10}$ eine lineare oder verzweigte, gesättigte oder ungesättigte Kohlenwasserstoffkette mit 5 bis 30 Kohlenstoffatomen darstellen;
- $R_2$, $R_4$, $R_7$ und $R_9$ eine lineare oder verzweigte, gesättigte oder ungesättigte Kohlenwasserstoffkette mit 1 bis 5 Kohlenstoffatomen darstellen;

die Gesamtzahl der Ethylenoxidmoleküle, die jeweils in den vorstehenden Formeln I, II und III durch k, l+m, n+p+q darstellt wird, eine ganze Zahl ist, so dass der HLB-Wert (hydrophiles-lipophiles Gleichgewicht) der Verbindungen zwischen 4 und 10, vorzugsweise zwischen 5 und 9 liegt.

**7.** Produkt nach Anspruch 6, **dadurch gekennzeichnet, dass** die vorstehende selbst-emulgierbare Zusammensetzung mindestens einen ethoxylierten Fettsäureester enthält, der der vorstehenden Formel I entspricht, in der $R_1$ ausgewählt ist aus den Resten der Palmitinsäure, Stearinsäure, Ricinolsäure, Ölsäure, Linolsäure und Linolensäure; $R_2$ eine Methylgruppe darstellt und k eine ganze Zahl zwischen 1 und 5, vorzugsweise gleich 2 ist.

**8.** Produkt nach Anspruch 6 oder 7, **dadurch gekennzeichnet, dass** die vorstehende selbst-emulgierbare Zusammensetzung mindestens einen ethoxylierten Fettsäureester enthält, der der vorstehenden Formel III entspricht, in der:

- $R_6$, $R_8$ und $R_{10}$ Kohlenwasserstoffketten darstellen, die den Fettsäureketten eines Pflanzenöls entsprechen;
- $R_7$ und $R_9$ eine Methylengruppe $CH_2$darstellen;
- n, p und q ganze Zahlen darstellen, so dass ihre Summe zwischen 3 und 30 liegt.

**9.** Produkt nach Anspruch 6 oder 7, **dadurch gekennzeichnet, dass** die vorstehende selbst-emulgierbare Zusammensetzung mindestens einen ethoxylierten Fettsäureester enthält, der der vorstehenden Formel III entspricht, in der:

- $R_6$, $R_8$ und $R_{10}$ Kohlenwasserstoffketten darstellen, die den Fettsäureketten von Rapsöl entsprechen;
- $R_7$ und $R_9$ eine Methylengruppe $CH_2$darstellen;
- n, p und q ganze Zahlen darstellen, so dass ihre Summe zwischen 3 und 30 liegt und bevorzugt gleich 20 ist.

**10.** Produkt nach Anspruch 6 oder 7, **dadurch gekennzeichnet, dass** die vorstehende selbst-emulgierbare Zusammensetzung mindestens einen ethoxylierten Fettsäureester enthält, der der vorstehenden Formel III entspricht, in der:

- $R_6$, $R_8$ und $R_{10}$ Kohlenwasserstoffketten darstellen, die den Fettsäureketten von Rizinusöl entsprechen;
- $R_7$ und $R_9$ eine Methylengruppe $CH_2$darstellen;
- n, p und q ganze Zahlen darstellen, so dass ihre Summe zwischen 5 und 7 liegt.

**11.** Produkt nach einem der Ansprüche 6 bis 10, **dadurch gekennzeichnet, dass** die vorstehende selbst-emulgierbare Zusammensetzung weiterhin ein biologisch abbaubares Lösungsmittel enthält, das mit den ethoxylierten Fettsäureestern mischbar ist, vorzugsweise ausgewählt aus Triglyceriden, Glykolen, Estern oder leichten Ketonen.

**12.** Produkt nach Anspruch 11, **dadurch gekennzeichnet, dass** die vorstehende selbst-emulgierbare Zusammensetzung aus:

50 bis 100 Gew.-% eines oder mehrerer ethoxylierter Fettsäureester wie in einem der Ansprüche 5 bis 8 definiert; und
0 bis 50 Gew.-% eines biologisch abbaubaren Lösungsmittels wie in Anspruch 9 definiert,

besteht.

**13.** Produkt nach einem der Ansprüche 6 bis 12, **dadurch gekennzeichnet, dass** es mindestens ein aktives Material oder einen aktiven Stoff in Verbindung mit einer selbst-emulgierbaren Zusammensetzung in einem entsprechenden Verhältnis von 1/99 bis 90/10 umfasst.

**14.** Produkt nach einem der Ansprüche 6 bis 13, **dadurch gekennzeichnet, dass** es weiterhin eine wässrige Phase aufweist und als stabile Emulsion vorliegt.

**15.** Verfahren zur Herstellung eines Produkts zur phytosanitären Behandlung oder zur pharmazeutischen Behandlung zur Verwendung in der Veterinär- oder Humanmedizin, das als stabile Emulsion vorliegt, **dadurch gekennzeichnet, dass** es aus Mischen praktisch ohne Energiezufuhr, z.B. durch Dispergieren durch langsames mechanisches Rühren, einer wässrigen Phase und einer selbst-emulgierbaren Zusammensetzung besteht, wobei die autoemulgierbare Zusammensetzung mindestens einen ethoxylierten Fettsäureester gemäß einer der folgenden Formeln umfasst:

(I) 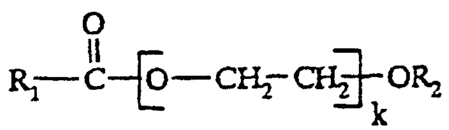

(II) 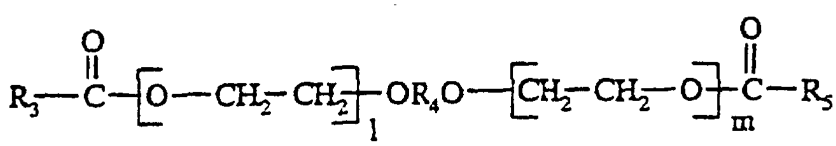

(III) 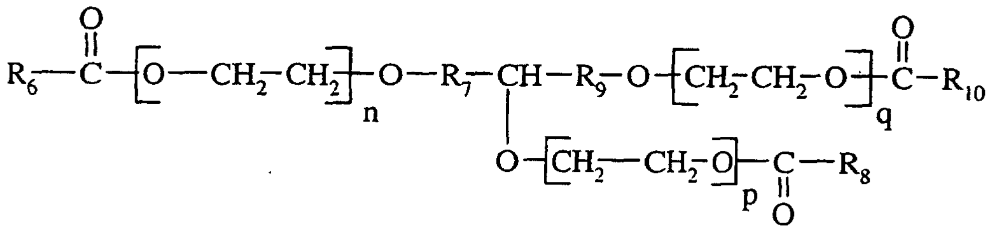

wobei:

- $R_1$, $R_3$, $R_5$, $R_6$, $R_8$ und $R_{10}$ eine lineare oder verzweigte, gesättigte oder ungesättigte Kohlenwasserstoffkette mit 5 bis 30 Kohlenstoffatomen darstellen;
- $R_2$, $R_4$, $R_7$ und $R_9$ eine lineare oder verzweigte, gesättigte oder ungesättigte Kohlenwasserstoffkette mit 1 bis 5 Kohlenstoffatomen darstellen;

die Gesamtzahl der Ethylenoxidmoleküle, die jeweils in den vorstehenden Formeln I, II und III durch k, l+m, n+p+q darstellt wird, eine ganze Zahl ist, so dass der HLB-Wert (hydrophiles-lipophiles Gleichgewicht) der Verbindungen zwischen 4 und 10, vorzugsweise zwischen 5 und 9 liegt.